# EUROPÄISCHE PATENTSCHRIFT

(11) **EP 3 203 910 B1**
(45) Veröffentlichungstag und Bekanntmachung des Hinweises auf die Patenterteilung: **21.11.2018**
(21) Anmeldenummer: 15816717.1
(22) Anmeldetag: 15.12.2015
(51) Int. Cl.: A61B 5/12

(54) **VORRICHTUNG UND VERFAHREN ZUR ERMITTLUNG DER FREQUENZ VON MINDESTENS EINEM DOMINANTEN TON EINES OHRGERÄUSCHS**
DEVICE AND METHOD FOR DETERMINING THE FREQUENCY OF AT LEAST ONE DOMINANT TONE OF A TINNITUS
DISPOSITIF ET PROCÉDÉ DE DÉTERMINATION DE LA FRÉQUENCE D'AU MOINS UN SON DOMINANT D'UN ACOUPHÈNE

(30) Priorität: 15.12.2014 DE 102014118674
(43) Veröffentlichungstag der Anmeldung: 16.08.2017
(73) Patentinhaber: Forschungszentrum Jülich GmbH, 52425 Jülich (DE)
(72) Erfinder: TASS, Peter Alexander, 83684 Tegernsee (DE); HAUPTMANN, Christian, 82319 Starnberg (DE)
(74) Vertreter: Müller-Boré & Partner Patentanwälte PartG mbB
(86) Internationale Anmeldenummer: PCT/EP2015/079720
(87) Internationale Veröffentlichungsnummer: WO 2016/096801

(56) Entgegenhaltungen:
- WO-A1-2014/109762
- US-A1- 2010 049 104
- US-A1- 2013 039 517

## Beschreibung

Die Erfindung betrifft eine Vorrichtung und ein Verfahren zur Ermittlung der Frequenz von mindestens einem dominanten Ton eines von einem Patienten wahrgenommenen Ohrgeräuschs.

Subjektiver Tinnitus ist die Wahrnehmung eines Tons oder Geräuschs ohne physikalische Ton- bzw. Geräuschquelle. Der subjektive Tinnitus entsteht im Gehirn des Patienten und kann nur von ihm wahrgenommen werden. Es handelt sich um eine Volkskrankheit, die bei vielen Patienten quälenden Charakter hat. Man unterscheidet zwischen einem tonalen Tinnitus, d. h. der Wahrnehmung eines oder mehrerer Töne, im Vergleich zu einem atonalen Tinnitus, d. h. der Wahrnehmung eines Geräuschs. Auch das beim atonalen Tinnitus wahrgenommene Geräusch kann jedoch einen oder mehrere dominante Töne enthalten.

Insbesondere für die Anwendung der akustischen "Coordinated Reset"-Neuromodulation (CR-Neuromodulation) zur Therapie des Tinnitus ist es außerordentlich wichtig, die Frequenzen, d. h. die Tonhöhen, der dominanten Töne möglichst genau zu ermitteln.

Bisher wurden zur Bestimmung der Tinnitus-Frequenz Methoden verwendet, bei denen dem Patienten typischerweise ein Vergleichston vorgespielt wurde und der Patient selbst die Frequenz und Amplitude des Vergleichstons so einstellen sollte, dass der Vergleichston dem Tinnitus-Ton entspricht. Diese Verfahren sind langwierig und führen bei einer relevanten Anzahl von Patienten nicht zu reproduzierbaren und damit verlässlichen Ergebnissen (vgl. E. M. Burns: A Comparison of Variability among Measurements of Subjective Tinnitus and Objective Stimuli, Audiology 23, 426-440 (1984)).

US 2010/049104 beschreibt eine Vorrichtung zur Behandlung von Tinnitus, umfassend Generatormittel, die ein Audiosignal erzeugen können, und Wandlermittel, die mit dem Generatormittel verbunden sind, um das Audiosignal zu reproduzieren. Die Vorrichtung umfasst auch Filtermittel, die zwischen den Generatormitteln und den Wandlermitteln angeordnet sind, um das Audiosignal zumindest in Übereinstimmung mit einem Intervall von Frequenzen um die dominante Frequenz des Tinnitus zu filtern und im Wesentlichen zu unterdrücken, um so ein Frequenzintervall (stummes Band) zu erhalten, in welchem kein Audiosignal wahrgenommen wird, welches eine ausgewählte Breite oder Amplitude um die dominante Frequenz des Tinnitus hat.

Der Erfindung liegt daher die Aufgabe zugrunde, eine Vorrichtung sowie ein Verfahren anzugeben, die es ermöglichen, die Frequenz von mindestens einem dominanten Ton eines von einem Patienten wahrgenommenen Ohrgeräuschs mit großer Genauigkeit und hoher Reproduzierbarkeit zu ermitteln.

Die der Erfindung zugrunde liegende Aufgabenstellung wird durch die Merkmale der unabhängigen Ansprüche gelöst. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:
- Fig. 1: eine schematische Darstellung einer Vorrichtung zur Ermittlung der Frequenz von mindestens einem dominanten Ton eines von einem Patienten wahrgenommenen Ohrgeräuschs;
- Fig. 2: eine Tabelle zur Eintragung von erhobenen Daten zur Ermittlung der Frequenz des mindestens einen dominanten Tons; und
- Fig. 3: eine mit beispielhaften Daten ausgefüllte Tabelle zur Ermittlung der Frequenz des mindestens einen dominanten Tons.

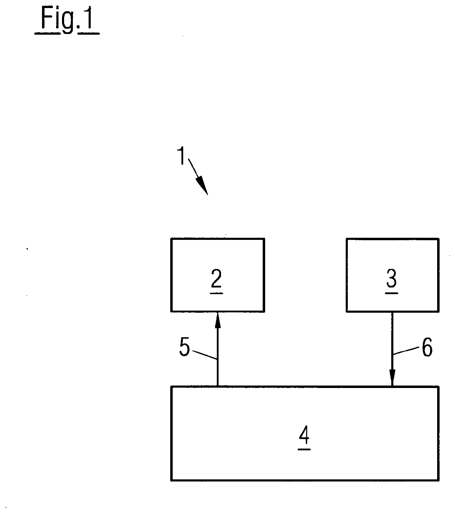

In Fig. 1 ist schematisch eine Vorrichtung 1 zur Ermittlung der Frequenz von mindestens einem dominanten Ton eines von einem Patienten wahrgenommenen Ohrgeräuschs dargestellt. Die Vorrichtung 1 besteht aus einem Tongenerator 2, einer Eingabeeinheit 3 und einer an den Tongenerator 2 sowie die Eingabeeinheit 3 gekoppelten Steuer- und Analyseeinheit 4.

Der Tongenerator 2 erzeugt Töne, die einem an Tinnitus erkrankten Patienten vorgespielt werden. Bei den Tönen handelt es sich um Vergleichstöne, die der Patient mit dem untersuchten dominanten Ton des von ihm wahrgenommenen Ohrgeräuschs vergleicht und dementsprechend bewertet.

Die von dem Patienten vorgenommene Bewertung der dargebotenen Töne wird in die Eingabeeinheit 3 eingegeben.

Die Steuer- und Analyseeinheit 4 führt während des Betriebs der Vorrichtung 1 eine Steuerung des Tongenerators 2 durch. Dazu übermittelt die Steuer- und Analyseeinheit 4 Steuersignale 5 an den Tongenerator 2. Ferner erhält die Steuer- und Analyseeinheit 4 Signale 6 von der Eingabeeinheit 3, mit denen der Steuer- und Analyseeinheit 4 die in die Eingabeeinheit 3 eingegebenen Bewertungen des Patienten bezüglich der dem Patienten dargebotenen Töne übermittelt werden.

Die Steuer- und Analyseeinheit 4 führt mit Hilfe des Tongenerators 2 und der Eingabeeinheit 3 verschiedene Untersuchungen durch, insbesondere eine Ähnlichkeitsmessung und einen paarweisen Tonvergleich.

Für die Ähnlichkeitsmessung steuert die Steuer- und Analyseeinheit 4 den Tongenerator 2 derart an, dass dieser dem Patienten nacheinander unterschiedliche Töne vorspielt. Nach jedem vorgespielten Ton bewertet der Patient den jeweiligen Ton hinsichtlich der Ähnlichkeit des Tons zu dem untersuchten dominanten Ton des von dem Patienten wahrgenommenen Ohrgeräuschs. Die Bewertung des Patienten wird in die Eingabeeinheit 3 eingegeben und an die Steuer- und Analyseeinheit 4 weitergeleitet.

Gemäß einer Ausgestaltung sind die Töne, die dem Patienten bei der Ähnlichkeitsmessung nacheinander vorgespielt werden, in ihrer Frequenz voneinander beabstandet, d. h., es wird für die Ähnlichkeitsmessung nicht eine Frequenzrampe hoch- oder runtergefahren.

Für den paarweisen Tonvergleich steuert die Steuer- und Analyseeinheit 4 den Tongenerator 2 derart an, dass dieser dem Patienten nacheinander Paare von Tönen vorspielt. Die Töne eines jeweiligen Paars werden dabei zeitversetzt und insbesondere nicht überlappend dargeboten. Nach jedem Paar bewertet der Patient, welcher Ton des Paars dem untersuchten dominanten Ton des Ohrgeräuschs ähnlicher ist. Die Bewertung des Patienten wird in die Eingabeeinheit 3 eingegeben und an die Steuer- und Analyseeinheit 4 weitergeleitet.

Die Steuer- und Analyseeinheit 4 kombiniert die Ergebnisse der Ähnlichkeitsmessung und des paarweisen Tonvergleichs und ermittelt daraus mindestens einen Ton, dessen Frequenz der Frequenz des untersuchten dominanten Tons des Ohrgeräuschs am ähnlichsten ist.

Bei dem von dem Patienten wahrgenommenen Ohrgeräusch, das mit der Vorrichtung 1 untersucht wird, kann es sich sowohl um einen tonalen als auch einen atonalen Tinnitus handeln. Im Fall eines tonalen Tinnitus nimmt der Patient mindestens einen Ton wahr, dessen Frequenz mit Hilfe der Vorrichtung 1 ermittelt wird. Im Fall eines atonalen Tinnitus nimmt der Patient ein Geräusch wahr, welches mindestens einen dominanten Ton enthalten kann, dessen Frequenz von der Vorrichtung 1 ermittelt wird. Falls der Patient mehrere dominante Töne wahrnimmt, deren Frequenz ermittelt werden soll, müssen die Ähnlichkeitsmessung und der paarweise Tonvergleich für jeden der dominanten Töne durchgeführt werden.

Die dem Patienten vorgespielten Töne lassen sich mathematisch durch eine Sinusschwingung beschreiben. Jeder Ton ist durch eine Tonhöhe, d. h. die Frequenz der Sinusschwingung, und eine Lautstärke, d. h. die Amplitude der Sinusschwingung, charakterisiert. Es können von dem Tongenerator 2 reine Töne erzeugt werden, deren Frequenzbandbreite idealerweise sehr gering ist. Die Frequenzbandbreite ist in der Praxis durch die die Töne erzeugenden Geräte limitiert. Ferner können für die hier beschriebenen Untersuchungen auch Töne verwendet werden, die eine vorbestimmte Frequenzbandbreite aufweisen.

Der die Töne erzeugende Tongenerator 2 kann einen Ohr- oder Kopfhörer oder einen anders ausgestalteten Lautsprecher aufweisen, wobei ein Ohrhörer ein im Ohrkanal platzierter Lautsprecher ist. Ferner kann der Tongenerator 2 eine elektronische Schaltung zur Erzeugung der Töne aufweisen. Die Töne können dem Patienten an einem Ohr oder beiden Ohren dargeboten werden.

Bei einseitigem Tinnitus werden die Töne von dem Tongenerator 2 am selben Ohr und/oder am gegenüberliegenden Ohr dargeboten. Bei beidseitigem Tinnitus werden die Töne am selben Ohr und/oder am gegenüberliegenden Ohr des dominanten Tons des Tinnitus appliziert. Bei beidseitig identisch und/oder zentral wahrgenommenem Tinnitus werden die Töne von dem Tongenerator 2 einseitig und/oder beidseitig dargeboten.

Die Steuer- und Analyseeinheit 4 kann zur Durchführung ihrer Aufgaben eine Recheneinheit, z. B. einen Prozessor oder Mikrocontroller, enthalten. Ferner kann es sich bei der Steuer- und Analyseeinheit 4 um einen Computer handeln. Die hierin beschriebenen Verfahren zur Ermittlung der Frequenz des mindestens einen dominanten Tons des von dem Patienten wahrgenommenen Ohrgeräuschs können als Software-Code in einem der Steuer- und Analyseeinheit 4 zugeordneten Speicher abgelegt sein.

Die Eingabeeinheit 3 kann z. B. die Tastatur eines Computers sein oder aber sie kann ein auf einem Bildschirm dargestelltes Menü umfassen, über welches sich Eingaben tätigen lassen. Typischerweise teilt der Patient einem Arzt oder Audiologen die Bewertungen der Töne mit, die der Arzt bzw. Audiologe daraufhin in die Eingabeeinheit 3 eingibt. Denkbar wäre aber auch, dass der Patient die Bewertungen selbst in die Eingabeeinheit 3 eingibt.

Gemäß einer Ausgestaltung ist die Steuer- und Analyseeinheit 4 dazu ausgelegt, als Ergebnis der Ähnlichkeitsmessung mindestens einen Ton zu ermitteln, dessen Frequenz der Frequenz des untersuchten dominanten Tons des Ohrgeräuschs gemäß der Ähnlichkeitsmessung am ähnlichsten ist. Ferner ermittelt die Steuer- und Analyseeinheit 4 als Ergebnis des paarweisen Tonvergleichs mindestens einen Ton, dessen Frequenz der Frequenz des untersuchten dominanten Tons des Ohrgeräuschs gemäß dem paarweisen Tonvergleich am ähnlichsten ist.

Bei der Ähnlichkeitsmessung kann für jeden dargebotenen Ton ein Ähnlichkeitswert ermittelt werden, welcher die Ähnlichkeit des jeweiligen Tons zu dem dominanten Ton des Ohrgeräuschs auf einer vorgegebenen Skala angibt. Anschließend kann der Ton mit dem höchsten Ähnlichkeitswert als derjenige Ton ausgewählt werden, der dem dominanten Ton des Ohrgeräuschs gemäß der Ähnlichkeitsmessung am ähnlichsten ist. Denkbar wäre auch, nicht nur einen, sondern mehrere Töne, die die höchsten Ähnlichkeitswerte aufweisen, am Ende der Ähnlichkeitsmessung auszuwählen.

Eine Ausgestaltung sieht vor, dass beim paarweisen Tonvergleich aus jedem dem Patienten vorgespielten Paar von Tönen derjenige Ton ausgewählt wird, der nach der Einschätzung des Patienten dem dominanten Ton des Ohrgeräuschs ähnlicher ist. Derartige Vergleiche werden für eine vorgegebene Anzahl von Paaren durchgeführt. Die Paare werden gemäß einer Ausgestaltung so gebildet, dass der über alle Paare gemittelte, insbesondere relative Frequenzabstand zwischen den beiden Tönen eines Paars so groß wie möglich ist. Folglich werden insbesondere nicht Paare aus einander benachbarten Tönen gebildet. Anschließend werden neue Paare aus den zuvor ausgewählten Tönen gebildet und die Töne dieser Paare werden von dem Patienten wieder mit dem dominanten Ton des Ohrgeräuschs verglichen und die ähnlicheren Töne werden ausgewählt. Dieses Verfahren kann entsprechend fortgesetzt werden, bis ein Ton verbleibt, der als derjenige Ton betrachtet wird, der dem dominanten Ton des Ohrgeräuschs gemäß dem paarweisen Tonvergleich am ähnlichsten ist. Auch hier kann vorgesehen sein, zum Abschluss der Untersuchungen nicht nur einen Ton, sondern mehrere Töne auszuwählen.

Die Ähnlichkeitsmessung und der paarweise Tonvergleich können unabhängig voneinander durchgeführt werden. Die Ergebnisse der beiden Untersuchungen können anschließend miteinander in geeigneter Weise kombiniert werden, um die Frequenz des dominanten Tons des von dem Patienten wahrgenommenen Ohrgeräuschs zu ermitteln.

Alternativ können die Ähnlichkeitsmessung und der paarweise Tonvergleich von der Steuer- und Analyseeinheit 4 in einer vorgegebenen Reihenfolge durchgeführt werden. Das Ergebnis der zuerst durchgeführten Untersuchung kann dann als Ausgangswert für die nachfolgende Untersuchung verwendet werden. Sofern beispielsweise die Ähnlichkeitsmessung als erste Untersuchung durchgeführt wird, können die Ergebnisse der Ähnlichkeitsmessung dazu verwendet werden, die Ausgangstöne des paarweisen Tonvergleichs festzulegen. Insbesondere können dem Patienten in diesem Fall während des paarweisen Tonvergleichs ausschließlich Töne vorgespielt werden, die innerhalb eines vorgegebenen Frequenzbereichs um die Frequenz des als Ergebnis der Ähnlichkeitsmessung ermittelten Tons mit der größten Ähnlichkeit zum dominanten Ton des Ohrgeräuschs liegen. Aus diesem Frequenzbereich können für den paarweisen Vergleich gemäß einer Ausgestaltung Töne ausgewählt werden und aus den ausgewählten Tönen können Paare derart gebildet werden, dass der über alle Paare gemittelte, insbesondere relative Frequenzabstand zwischen den beiden Tönen eines Paars so groß wie möglich ist.

Im umgekehrten Fall, in dem die Ähnlichkeitsmessung auf den paarweisen Tonvergleich folgt, kann das Ergebnis des paarweisen Tonvergleichs als Ausgangswert für die Ähnlichkeitsmessung herangezogen werden.

Die Ähnlichkeitsmessung und der paarweise Tonvergleich können den Startwert oder Startbereich der jeweils anderen Untersuchung liefern. Sie können aber auch durch redundante Testung verlässlichere Informationen liefern.

Sowohl für die Ähnlichkeitsmessung als auch den paarweisen Tonvergleich kann jeweils ein Frequenzabstand zwischen benachbarten Tönen vorgegeben sein. Die zuerst ausgeführte Untersuchung weist dann typischerweise einen größeren Frequenzabstand auf als die nachfolgend durchgeführte Untersuchung. Für den Fall, dass erst die Ähnlichkeitsmessung und danach der paarweise Tonvergleich vorgenommen werden, liegen bezüglich ihrer Frequenz benachbarte Töne des paarweisen Tonvergleichs enger beieinander als benachbarte Töne der Ähnlichkeitsmessung.

Gemäß einer Weiterbildung der vorstehenden Ausgestaltung kann vor der Ähnlichkeitsmessung und dem paarweisen Tonvergleich eine grobe Eingrenzung des Tinnitus mit Hilfe von vorgegebenen Testtönen durchgeführt werden. Dazu können dem Patienten nacheinander die vorgegebenen Testtöne vorgespielt werden und der Patient gibt an, ob die Frequenz des jeweiligen Testtons größer oder kleiner als die Frequenz des dominanten Tons des Ohrgeräuschs ist. Der Frequenzabstand zwischen benachbarten Testtönen ist bei diesem Test größer als der Frequenzabstand zwischen benachbarten Tönen bei der Ähnlichkeitsmessung und dem paarweisen Tonvergleich. Weiterhin kann nach der Ähnlichkeitsmessung und dem paarweisen Tonvergleich eine Feinjustierung des Tons vorgenommen werden, welcher aus der Ähnlichkeitsmessung und dem paarweisen Tonvergleich als der Ton hervorgegangen ist, der dem mindestens einen dominanten Ton des Ohrgeräuschs am ähnlichsten ist. Die Feinjustierung kann z. B. in den Grenzen von ± 10% um den ermittelten Ton herum erfolgen. Die Frequenzauflösung bei der Feinjustierung ist kleiner als bei der Ähnlichkeitsmessung und dem paarweisen Tonvergleich.

Bei der Ähnlichkeitsmessung und/oder dem paarweisen Tonvergleich kann die Ähnlichkeit der Töne vom Patienten mittels eines psychophysischen Ähnlichkeitsmaßes, insbesondere hinsichtlich der Tonhöhe und/oder der Lautheit und/oder dem Klangcharakter, bewertet werden.

Gemäß einer Ausgestaltung besteht die Ähnlichkeitsmessung aus einer Abfolge von Ähnlichkeitsmessungen, wobei die Reihenfolge der einzelnen Ähnlichkeitstests von den jeweiligen Vorergebnissen abhängt. Insbesondere kann ein jeweiliger Ton, der dem Patienten für einen Ähnlichkeitstest vorgespielt wird, von der von dem Patienten vorgenommenen Bewertung eines oder mehrerer zuvor vorgespielter Töne abhängen.

Analog kann eine Tonvergleichsmessung aus einer Abfolge von paarweisen Tonvergleichen bestehen, deren Reihenfolge von den jeweiligen Vorergebnissen abhängt. Insbesondere kann ein jeweiliges Paar von Tönen, das dem Patienten für den paarweisen Vergleich vorgespielt wird, von der von dem Patienten vorgenommenen Bewertung eines oder mehrerer zuvor vorgespielter Paare von Tönen abhängen.

Außerdem müssen derartige Abfolgen von einzelnen Ähnlichkeitsmessungen und Tonvergleichen nicht separat durchgeführt werden, sondern können auch gemischt und bezüglich ihrer Reihenfolge in Abhängigkeit von den jeweiligen Vorergebnissen durchgeführt werden.

Die Amplitude, d. h. die Lautstärke, der dem Patienten vorzuspielenden Töne lässt sich ermitteln, indem dem Patienten ein Testton vorgespielt wird und der Patient die Amplitude dieses Tons so einstellt, dass der vorgespielte Ton genauso laut ist wie der vom Patienten wahrgenommene dominante Ton des Ohrgeräuschs. Anschließend kann die Amplitude der für die Ähnlichkeitsmessung und/oder den paarweisen Tonvergleich vorgespielten Töne an die vom Patienten eingestellte Amplitude angepasst werden.

Weiterhin lässt sich die Amplitude der dem Patienten vorzuspielenden Töne ermitteln, indem die Amplituden von zwei unterschiedlichen Testtönen vom Patienten so eingestellt werden, dass sie jeweils genauso laut sind wie der dominante Ton des Ohrgeräuschs. Die zwei Testtöne können dann als Stützstellen einer Inter- oder Extrapolation verwendet werden, welche es erlaubt, die Amplitude eines für die Ähnlichkeitsmessung und/oder den paarweisen Tonvergleich vorgespielten jeweiligen Tons zu ermitteln.

Außerdem kann die Amplitude eines für die Ähnlichkeitsmessung und/oder den paarweisen Tonvergleich vorgespielten jeweiligen Tons durch einen Vergleich mit einem (insbesondere für alle derartigen Test gewählten) Referenzton bestimmt werden.

Im Ergebnis können die dem Patienten für die Ähnlichkeitsmessung und/oder den paarweisen Tonvergleich vorgespielten Töne jeweils nahezu die gleiche Lautstärke wie der vom Patienten wahrgenommene dominante Ton des Ohrgeräuschs aufweisen. Die Erfindung erlaubt aber auch, dass hiervon abgewichen wird, und die Lautstärke der dem Patienten für die Ähnlichkeitsmessung und/oder den paarweisen Tonvergleich vorgespielten Töne größer oder geringer als die Lautstärke des vom Patienten wahrgenommenen dominanten Ton des Ohrgeräuschs ist.

Vor der Durchführung der Ähnlichkeitsmessung und des paarweisen Tonvergleichs ist es sinnvoll, den Frequenzbereich, aus dem die Testtöne gewählt werden, grob einzugrenzen. Dazu können dem Patienten nacheinander Töne vorgespielt werden und der Patient gibt jeweils an, ob die Frequenz des Tons größer oder kleiner als die Frequenz des dominanten Tons des Ohrgeräuschs ist.

Als Ergebnis wird ein eingegrenzter, aber noch vergleichsweise großer Frequenzbereich ermittelt, in welchem sich die Frequenz des dominanten Tons des Ohrgeräuschs befindet. Bei der anschließenden Ähnlichkeitsmessung und dem paarweisen Tonvergleich werden die dem Patienten vorgespielten Töne oder Paare von Tönen ausschließlich aus dem ermittelten Frequenzbereich ausgewählt.

Ein Verfahren zur Ermittlung der Frequenz von mindestens einem dominanten Ton eines von einem Patienten wahrgenommenen Ohrgeräuschs umfasst, dass eine Ähnlichkeitsmessung durchgeführt wird, bei welcher dem Patienten nacheinander Töne vorgespielt werden und der Patient nach dem Vorspielen eines jeweiligen Tons die Ähnlichkeit des Tons zu dem mindestens einen dominanten Ton des Ohrgeräuschs bewertet. Ferner wird ein paarweiser Tonvergleich durchgeführt, bei welchem dem Patienten nacheinander Paare von Tönen vorgespielt werden und der Patient nach dem Vorspielen eines jeweiligen Paars bewertet, welcher Ton des Paars dem mindestens einen dominanten Ton des Ohrgeräuschs ähnlicher ist. Die Ergebnisse der Ähnlichkeitsmessung und des paarweisen Tonvergleichs werden kombiniert und daraus wird mindestens ein Ton ermittelt, dessen Frequenz der Frequenz des mindestens einen dominanten Tons des Ohrgeräuschs am ähnlichsten ist. Das vorstehende Verfahren kann von einem Arzt oder Audiologen durchgeführt werden. Das Verfahren kann alle vorstehend beschriebenen Ausgestaltungen aufweisen. Für die Minimalvariante des Verfahrens reicht als technisches Hilfsmittel ein Tongenerator zur Erzeugung der Testtöne aus.

Eine Software wird in einem Datenverarbeitungssystem, insbesondere in der Steuer- und Analyseeinheit 4, ausgeführt. Die Software bewirkt, dass Steuersignale zum Ansteuern eines Tongenerators zum Vorspielen von Tönen erzeugt werden, von einer Eingabeeinheit Bewertungen der vorgespielten Töne durch einen Patienten entgegengenommen werden, der Tongenerator für eine Ähnlichkeitsmessung derart angesteuert wird, dass dieser nacheinander Töne vorspielt, und nach dem Vorspielen eines jeweiligen Tons von der Eingabeeinheit eine Bewertung des Patienten bezüglich der Ähnlichkeit des Tons zu mindestens einem dominanten Ton eines von dem Patienten wahrgenommenen Ohrgeräuschs entgegengenommen wird. Ferner bewirkt die Software, dass der Tongenerator für einen paarweisen Tonvergleich derart angesteuert wird, dass dieser nacheinander Paare von Tönen vorspielt, und nach dem Vorspielen eines jeweiligen Paars von der Eingabeeinheit eine Bewertung des Patienten entgegengenommen wird, welcher Ton des Paars dem mindestens einen dominanten Ton des Ohrgeräuschs ähnlicher ist, und die Ergebnisse der Ähnlichkeitsmessung und des paarweisen Tonvergleichs kombiniert werden und daraus mindestens ein Ton ermittelt wird, dessen Frequenz der Frequenz des mindestens einen dominanten Tons des Ohrgeräuschs am ähnlichsten ist. Die Software kann alle vorstehenden, im Zusammenhang mit der Vorrichtung 1 beschriebenen Ausgestaltungen aufweisen.

Als weiteres Ausführungsbeispiel wird im Folgenden ein Verfahren beschrieben, mit welchem sich die Frequenz des mindestens einen dominanten Tons des vom Patienten wahrgenommenen Ohrgeräuschs ermitteln lässt. Das nachfolgend beschriebene Verfahren kann mit Hilfe der in Fig. 1 dargestellten Vorrichtung 1 durchgeführt werden. Die Anweisungen an den Patienten zur Durchführung des Verfahrens können beispielsweise auf einem Bildschirm erscheinen, es kann aber auch vorgesehen sein, dass ein Arzt oder Audiologe dem Patienten die Anweisungen erteilt.

Vor der Durchführung der eigentlichen Messungen soll dem Patienten die Bedeutung von Amplitude, d. h. Lautstärke, und Tonhöhe, d. h. Frequenz, erläutert werden, um den Patienten in die Lage zu versetzen, Töne unterscheiden zu können.

Zur Erklärung der Amplitude wird dem Patienten ein Ton von 1.000 Hz vorgespielt. Anschließend wird die Amplitude erhöht. Zur Erklärung der Tonhöhe wird zuerst ein Ton von 1.000 Hz vorgespielt und im Anschluss ein Ton von 1.800 Hz. Dem Patienten wird erklärt, dass der zweite Ton (1.800 Hz) eine höhere Tonhöhe besitzt als der erste Ton (1.000 Hz). Danach werden beide Töne erneut abgespielt.

Zur Verständniskontrolle werden ein erster Ton von 2.000 Hz und ein zweiter von 2.400 Hz vorgespielt. Der Patient wird gefragt, welcher Ton höher ist. Dieser Test wird zwei Mal mit 3.000 Hz und 1.800 Hz wiederholt.

Ferner werden ein erster leiser Ton von 2.700 Hz und ein zweiter lauterer Ton von 2.700 Hz abgespielt, wobei die Lautstärke beider Töne oberhalb der Hörschwelle liegt. Der Patient wird gefragt, welcher Ton lauter ist und ob der Patient einen Unterschied der Tonfrequenz wahrnimmt oder nicht.

Ferner werden drei Töne von 14.000 Hz, 11.200 Hz und 8.900 Hz in absteigender Reihenfolge abgespielt. Der Patient wird gefragt, ob die Töne höher oder niedriger werden oder ähnlich sind. Sollte der Patient die höchsten Töne nicht unterscheiden können, werden diese Töne von den folgenden Arbeitsschritten ausgenommen.

Wenn die Verständniskontrolle beim Patienten erfolgreich verlaufen ist, kann mit der Frequenz-Ermittlung des Ohrgeräuschs begonnen werden. Falls nicht, werden die vorstehenden Schritte der Erklärungsphase erneut durchgeführt.

Die folgenden Arbeitsschritte werden für das linke und das rechte Ohr separat durchgeführt. Es wird mit der Seite begonnen, an welcher der Patient das Ohrgeräusch am stärksten wahrnimmt. Ferner wird jeder Vergleichston beispielsweise für wenigstens 2 Sekunden abgespielt.

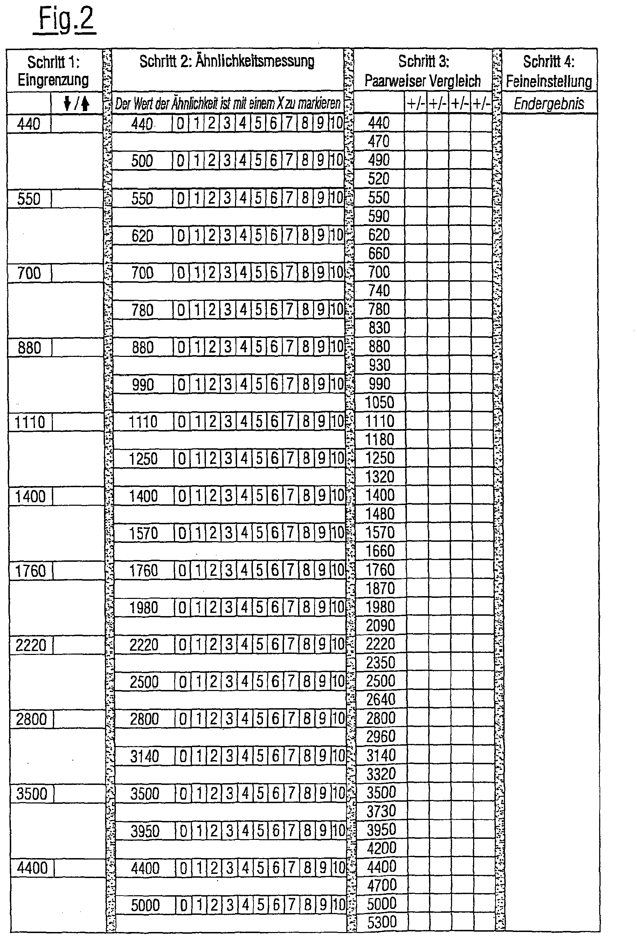

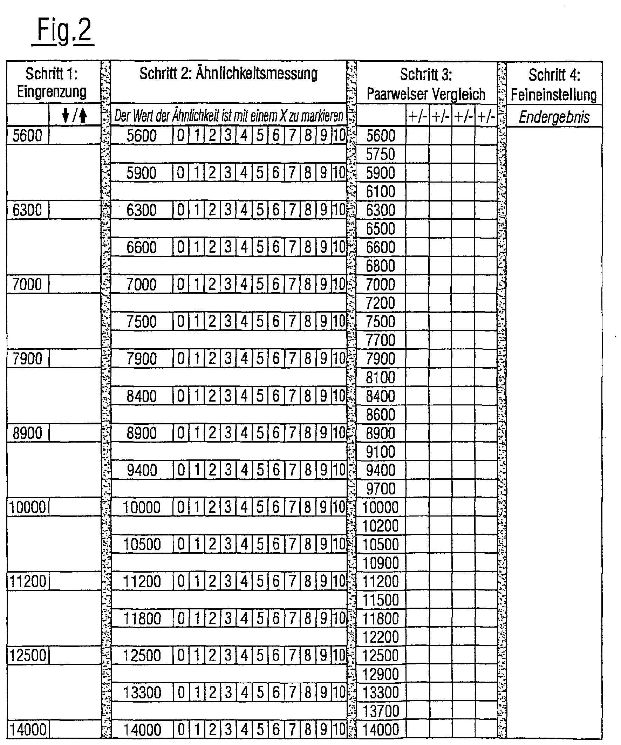

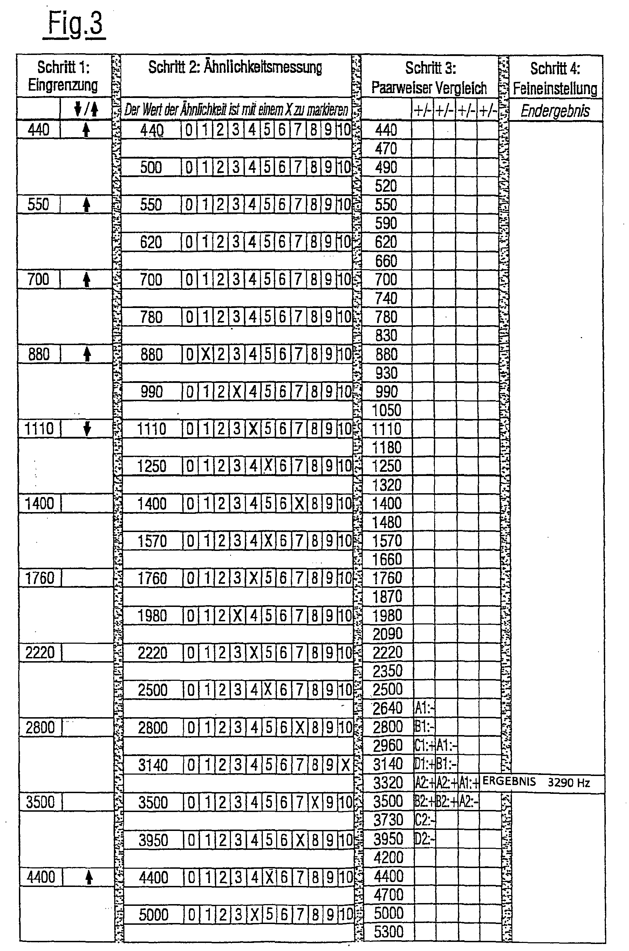

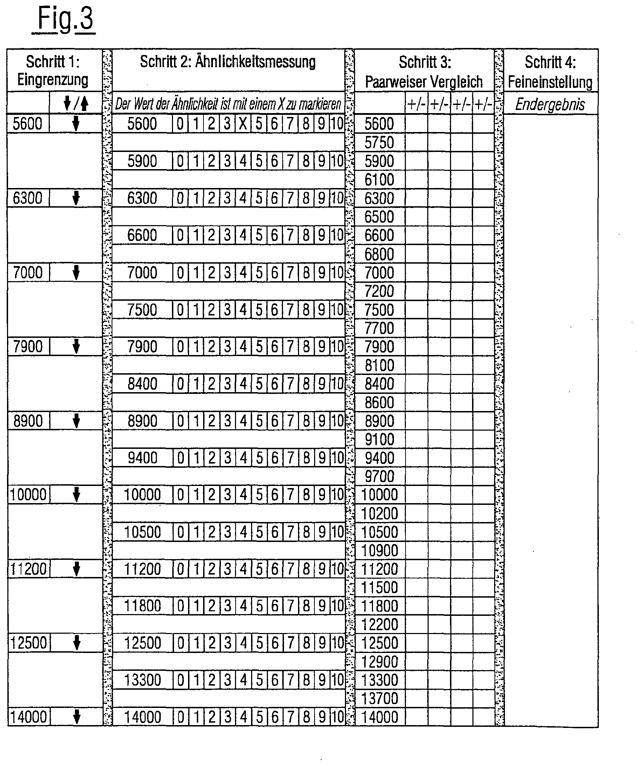

Die Ergebnisse der Arbeitsschritte können in eine Tabelle eingetragen werden, wie sie beispielhaft in Fig. 2 dargestellt ist. Die Tabelle kann vom Arzt oder Audiologen ausgefüllt werden, sie kann aber auch in einem Speicher, auf den die Steuer- und Analyseeinheit 4 Zugriff hat, abgelegt werden, so dass die entsprechenden Daten von der Steuer- und Analyseeinheit 4 in die Tabelle eingetragen werden können. Zum leichteren Verständnis der Untersuchungen zeigt Fig. 3 die mit den im Folgenden beschriebenen Beispieldaten ausgefüllte Tabelle.

In Schritt 1 erfolgt eine grobe Eingrenzung der Frequenz des untersuchten dominanten Tons des Ohrgeräuschs. Es empfiehlt sich, die grobe Eingrenzung bei der Erstuntersuchung nicht durchzuführen, um das gesamte Spektrum des Ohrgeräuschs des jeweiligen Patienten erfassen zu können. Die Kenntnis des gesamten Spektrums ist sehr hilfreich, sobald ein erster dominanter Ton des Ohrgeräuschs durch eine Therapie, z. B. eine CR-Neuromodulation, reduziert ist und ein zweiter dominanter Ton des Ohrgeräuschs ermittelt werden muss.

Ziel dieses Schritts ist, einen ersten groben Eindruck von der Frequenz des dominanten Tons des Ohrgeräuschs des jeweiligen Patienten zu gewinnen. Der Patient soll eine bestimmte Anzahl von Testtönen hören, wobei vom Patienten abgefragt werden soll, ob diese ober- oder unterhalb der Frequenz des dominanten Tons des Ohrgeräuschs des Patienten liegen. Für diesen Schritt werden 16 Töne verwendet, die die Frequenzbereiche zwischen 440 Hz und 14.000 Hz abdecken. Die Schrittweite ist ein Drittel einer Oktave (ca. 25% relative Differenz zu den nächstgelegenen Testtönen).

Zunächst werden dem Patienten das Verfahren und dessen Ablauf erläutert.

Zur Eingrenzung des Spektrums nach unten wird mit der niedrigsten Frequenz von 440 Hz begonnen und der Patient wird aufgefordert, die Amplitude des vorgespielten Tons so anzupassen, dass der Ton ebenso laut wie der dominante Ton seines Ohrgeräuschs ist. Der Patient wird gefragt, ob der dominante Ton seines Ohrgeräuschs niedriger oder höher als die Frequenz des vorgespielten Tons ist. Falls der dominante Ton des Ohrgeräuschs höher ist, wird ein nach oben zeigender Pfeil in die für Schritt 1 vorgesehene Spalte der in Fig. 2 gezeigten Tabelle eingetragen. Falls der dominante Ton des Ohrgeräuschs niedriger ist, wird ein nach unten zeigender Pfeil in die Tabelle eingetragen. Der Vorgang wird mit der nächsten Frequenz von 550 Hz und auch der darauffolgenden Frequenz von 700 Hz wiederholt.

Wenn der Patient mitteilt, dass der dominante Ton des Ohrgeräuschs niedriger als ein Ton aus dem unteren Spektrum ist (440 Hz, 550 Hz, 700 Hz,...), d. h., wenn der Pfeil die Richtung umkehrt (von aufwärts nach abwärts), wird der Test für das untere Spektrum beendet.

Zur Eingrenzung des Spektrums nach oben wird mit der höchsten Frequenz von 14.000 Hz begonnen. Der Ton wird dem Patienten vorgespielt und der Patient wird gebeten, die Amplitude des Tons so anzupassen, dass die Lautstärke des externen Tons mit der Amplitude des dominanten Tons des Ohrgeräuschs übereinstimmt. Der Patient wird gefragt, ob die Frequenz des dominanten Tons des Ohrgeräuschs niedriger oder höher als die Frequenz des externen Tons ist. Falls der dominante Ton des Ohrgeräuschs höher ist, wird ein Aufwärtspfeil in die für Schritt 1 vorgesehene Spalte der in Fig. 2 gezeigten Tabelle eingetragen. Falls der dominante Ton des Ohrgeräuschs niedriger ist, wird ein Abwärtspfeil in die Tabelle eingefügt.

Der Vorgang wird wie vorstehend beschrieben mit der zweithöchsten Frequenz von 12.500 Hz und anschließend der dritthöchsten Frequenz von 11.200 Hz wiederholt.

Wenn der Patient feststellt, dass der dominante Ton des Ohrgeräuschs höher ist als der Ton aus dem oberen Spektrum (14.000 Hz, 12.500 Hz, 11.200 Hz,...), d. h., wenn sich die Pfeilrichtung umkehrt (von abwärts nach aufwärts), wird der Test für das obere Spektrum beendet.

Als Beispiel für diese Messung sind in der in Fig. 3 dargestellten Tabelle beispielhafte Pfeile für den Frequenz-Vergleich eingetragen.

Als Ergebnis liefert der Schritt 1 zwei Zahlen, die untere Begrenzung und die obere Begrenzung. In dem Beispiel aus Fig. 3 beträgt die untere Begrenzung 880 Hz und die obere Begrenzung 5.600 Hz, d. h., es wird die Frequenz genau vor dem Pfeilrichtungswechsel ermittelt.

Nachdem in Schritt 1 das Frequenzspektrum eingegrenzt wurde, wird in Schritt 2 eine Ähnlichkeitsmessung durchgeführt. In diesem Schritt werden Testtöne abgespielt, wobei der Patient deren Ähnlichkeit zur Frequenz des dominanten Tons seines Ohrgeräuschs angeben soll. Hierfür steht eine beispielhafte Skala von 0 bis 10 zur Verfügung - 0 bedeutet keine Ähnlichkeit in dem Sinne, dass beide Töne komplett verschieden sind, und 10 bedeutet große Ähnlichkeit im Sinne, dass beide Töne identisch sind. Bevor die Ähnlichkeit bewertet werden kann, ist die Amplitude des Testtons auf die gleiche Amplitude des dominanten Tons des Ohrgeräuschs des Patienten einzustellen. Die Schrittweite ist entweder ein ganzer Ton (unterer Teil der Frequenzskala, etwa 12% relative Differenz) oder ein halber Ton (oberer Teil der Frequenzskala, etwa 6% relative Differenz).

Zunächst werden dem Patienten das Verfahren und dessen Ablauf erläutert.

Es wird anschließend mit der niedrigsten Frequenz innerhalb der Eingrenzung (880 Hz) begonnen. Der Ton wird abgespielt und der Patient wird gebeten, die Amplitude des Tons so einzustellen, dass der externe Ton genauso laut ist wie der dominante Ton des Ohrgeräuschs des Patienten. Der Patient wird gefragt, wie ähnlich dieser externe Ton dem dominanten Ton seines Ohrgeräuschs ist. Der Patient wird gebeten, dies anhand der Skala von 0 bis 10 anzugeben, wobei 0 keinerlei Ähnlichkeit und 10 vollständige Übereinstimmung bedeuten. Die Bewertung des Patienten wird in das entsprechende Kästchen der Tabelle mit einem "X" eingetragen.

Der vorstehende Vorgang wird mit einem anderen Ton innerhalb der Eingrenzung, z. B. 2.800 Hz, wiederholt. Es wird solange fortgefahren, bis für alle Töne Ähnlichkeitswerte ermittelt und in die Tabelle eingetragen sind.

Falls der Patient sich im Hinblick auf einen Ähnlichkeitswert unsicher ist, wird die Ähnlichkeitsmessung für den entsprechenden Ton wiederholt und der alte Ähnlichkeitswert wird gegebenenfalls durch einen neuen Ähnlichkeitswert ersetzt.

Ein Beispiel für eine solche Ähnlichkeitsmessung ist in Fig. 3 dargestellt, dort sind beispielhafte Ähnlichkeitswerte mit "X" eingetragen. In diesem Beispiel wurden Testtöne innerhalb der in Schritt 1 ermittelten Eingrenzung von 880 Hz bis 5.600 Hz verwendet. Alle anderen Töne, die kleiner als 880 Hz oder größer als 5.600 Hz sind, sind ausgestrichen. Für jeden Testton ist die Ähnlichkeit mit einem "X" angegeben. In dem vorliegenden Beispiel besitzt der Testton von 3.140 Hz den größten Ähnlichkeitswert mit dem dominanten Ton des Ohrgeräuschs. Die Bestimmung dieser Frequenz ist das Ergebnis des Schritts 2.

In dem nachfolgenden Schritt 3 wird ein paarweiser Tonvergleich durchgeführt. In diesem Arbeitsschritt werden dem Patienten auf einer stärker differenzierteren Skala Tonpaare vorgespielt, wobei der Patient entscheiden soll, welcher der jeweiligen zwei Töne dem dominanten Ton des von ihm wahrgenommenen Ohrgeräuschs in Bezug auf die Tonhöhe mehr ähnelt. Hierfür werden beispielsweise acht Töne ausgewählt, die um den Ton mit dem in Schritt 2 ermittelten höchsten Ähnlichkeitswert (in der Beispieltabelle aus Fig. 3 ist dies der Ton von 3.140 Hz) herum gruppiert sind, und dann zu Paaren zusammengestellt. Die Schrittweite ist entweder ein Halbton (unterer Teil der Frequenzskala, ungefähr 6% relative Differenz) oder ein Viertelton (oberer Teil der Frequenzskala, ungefähr 3% relative Differenz).

Zunächst werden dem Patienten das Verfahren und dessen Ablauf erläutert.

In einem ersten paarweisen Tonvergleich werden acht Töne paarweise miteinander verglichen, die in der Tabelle aus Fig. 3 in der ersten Spalte von Schritt 3 mit A1, B1, C1, D1, A2, B2, C2, D2 bezeichnet sind und aus dem Frequenzbereich von 2.640 Hz bis 3.950 Hz stammen.

Das erste Tonpaar wird aus dem ersten und fünften Ton geformt, welche in Fig. 3 mit A1 bzw. A2 bezeichnet sind. Der Ton A1 (2.640 Hz) wird abgespielt, und der Patient wird aufgefordert, die Amplitude dieses Tons so einzustellen, dass der externe Ton ebenso laut ist wie der dominante Ton des von ihm wahrgenommenen Ohrgeräuschs. Dieser Ton wird mit "Ton 1" benannt.

Ferner wird der Ton A2 (3.320 Hz) dem Patienten vorgespielt, und der Patient wird erneut aufgefordert, die Amplitude dieses Tons so einzustellen, dass der Ton ebenso laut ist wie der dominante Ton des Ohrgeräuschs. Dieser Ton wird mit "Ton 2" benannt.

Es wird zwischen den Tönen gewechselt, wobei die Töne stets mit "Ton 1" bzw. "Ton 2" benannt werden. Der Patient wird gefragt, welcher Ton dem dominanten Ton seines Ohrgeräuschs mehr ähnelt. Der ähnlichere Ton wird in der Tabelle mit "+" und der weniger ähnliche mit "-" markiert.

Das zweite Paar wird aus dem zweiten und dem sechsten Ton geformt, welche in Fig. 3 mit B1 bzw. B2 bezeichnet sind. Das zweite Tonpaar wird in gleicher Weise wie das erste Tonpaar untersucht.

Anschließend wird mit dem dritten Tonpaar (C1 und C2) und vierten Tonpaar (D1 und D2) wie oben beschrieben fortgefahren.

Im ersten paarweisen Tonvergleich wurden vier Töne ermittelt, die in der ersten Spalte von Schritt 3 in Fig. 3 mit "+" markiert sind. Diese Töne werden dazu verwendet, die Tonpaare für den zweiten paarweisen Tonvergleich zu formen. Alle anderen Töne sind ausgestrichen.

Das erste Tonpaar des zweiten paarweisen Tonvergleichs wird zusammengestellt aus den "Gewinnern" aus A1/A2 und C1/C2 des ersten paarweisen Tonvergleichs. In der Beispieltabelle von Fig. 3 sind dies die Töne mit 2.960 Hz und 3.320 Hz. Die Untersuchung dieses Tonpaars erfolgt in gleicher Weise wie oben beschrieben.

Das zweite Tonpaar des zweiten paarweisen Tonvergleichs setzt sich zusammen aus den "Gewinnern" aus B1/B2 und D1/D2 des ersten paarweisen Tonvergleichs. In der Beispieltabelle von Fig. 3 sind dies die Töne mit 3.140 Hz und 3.500 Hz. Die Untersuchung dieses Tonpaars erfolgt in gleicher Weise wie oben beschrieben.

Als Ergebnis des zweiten paarweisen Tonvergleichs wurden zwei Töne mit "+" markiert. In der Beispieltabelle von Fig. 3 sind dies die Töne mit 3.320 Hz und 3.500 Hz. Diese beiden Töne bilden das Paar für den dritten und letzten paarweisen Tonvergleich. Die Untersuchung des Tonpaars wird wieder analog zu den vorgehergehenden Vergleichsuntersuchungen durchgeführt.

Der Gewinner aus dem letzten Vergleich, in der in Fig. 3 dargestellten Beispieltabelle ist dies der Ton mit 3.320 Hz, ist das Ergebnis aus Schritt 3.

Es ist weiterhin denkbar, in Schritt 3 von dem vorstehend beschriebenen Verfahren abzuweichen. Anstatt den Patienten zwischen zwei abgespielten Tönen, dem Tonpaar, entscheiden zu lassen, kann auch eine Ähnlichkeitsmessung für jeden der zwei Töne wie in Schritt 2 durchgeführt werden. Der Ton mit dem jeweils höchsten Ähnlichkeitswert wird dann dazu verwendet, das nächste Paar zu bilden und der Ton mit dem jeweils niedrigeren Wert scheidet für die nächste Vergleichsstufe aus.

In Schritt 4 wird schließlich eine Feineinstellung des externen Tons innerhalb eng gesetzter Grenzen vorgenommen, um eine größtmögliche Annäherung an den dominanten Ton des Ohrgeräuschs zu erreichen. Innerhalb eng gesetzter Grenzen bedeutet hierbei ± 5%. Dementsprechend kann bei dem vorliegenden Beispiel eine Feinabstimmung innerhalb des Intervalls von 3.140 bis 3.500 Hz, welches die Frequenzen der zwei benachbarten Töne aus Schritt 3 sind, vorgenommen werden. Die Schrittweite innerhalb dieses Intervalls ist flexibel (ca. 1% Genauigkeit).

Zunächst werden dem Patienten das Verfahren und dessen Ablauf erläutert.

Daraufhin wird ein Ton mit der in Schritt 3 ermittelten Frequenz abgespielt (in der Tabelle aus Fig. 3 ist dies 3.320 Hz), und der Patient wird aufgefordert, die Amplitude dieses Tons so einzustellen, dass sie der Amplitude des von ihm wahrgenommenen dominanten Tons des Ohrgeräuschs entspricht.

Anschließend kann der Patient, beispielsweise mit Hilfe eines Drehknopfs, die Frequenz des Tons innerhalb der gesetzten Grenzen einstellen. Der Patient soll die Feinabstimmung beenden, sobald der externe Ton optimal auf die Frequenz des dominanten Tons des Ohrgeräuschs eingestellt ist. Diese Frequenz ist das Ergebnis des hier beschriebenen Verfahrens und wird in die Tabelle unter Schritt 4 eingetragen. In der in Fig. 3 dargestellten Beispieltabelle beträgt die in Schritt 4 ermittelte Frequenz 3.290 Hz.

## Patentansprüche

1. Vorrichtung (1) zur Ermittlung der Frequenz von mindestens einem dominanten Ton eines von einem Patienten wahrgenommenen Ohrgeräuschs, umfassend
- einen Tongenerator (2) zum Vorspielen von Tönen,
- eine Eingabeeinheit (3) zur Eingabe von Bewertungen der vorgespielten Töne durch den Patienten, und
- eine an den Tongenerator (2) und die Eingabeeinheit (3) gekoppelte Steuer- und Analyseeinheit (4), welche dazu ausgelegt ist, eine Ähnlichkeitsmessung und einen paarweisen Tonvergleich durchzuführen, wobei
- die Steuer- und Analyseeinheit (4) für die Ähnlichkeitsmessung den Tongenerator (2) derart ansteuert, dass dieser nacheinander Töne vorspielt, und die Steuer- und Analyseeinheit (4) nach dem Vorspielen eines jeweiligen Tons die in die Eingabeeinheit (3) eingegebene Bewertung des Patienten bezüglich der Ähnlichkeit des Tons zu dem mindestens einen dominanten Ton des Ohrgeräuschs entgegennimmt,
- die Steuer- und Analyseeinheit (4) für den paarweisen Tonvergleich den Tongenerator (2) derart ansteuert, dass dieser nacheinander Paare von Tönen vorspielt, und die Steuer- und Analyseeinheit (4) nach dem Vorspielen eines jeweiligen Paars die in die Eingabeeinheit (3) eingegebene Bewertung des Patienten entgegennimmt, weleher Ton des Paars dem mindestens einen dominanten Ton des Ohrgeräuschs ähnlicher ist, **dadurch gekennzeichnet, dass**
- die Steuer- und Analyseeinheit (4) die Ergebnisse der Ähnlichkeitsmessung und des paarweisen Tonvergleichs kombiniert und daraus mindestens einen Ton ermittelt, dessen Frequenz der Frequenz des mindestens einen dominanten Tons des Ohrgeräuschs am ähnlichsten ist,
- die Steuer- und Analyseeinheit (4) dazu ausgelegt ist, als Ergebnis der Ähnlichkeitsmessung einen Ton zu ermitteln, dessen Frequenz der Frequenz des mindestens einen dominanten Tons des Ohrgeräuschs gemäß der Ähnlichkeitsmessung am ähnlichsten ist, und
- die Steuer- und Analyseeinheit (4) dazu ausgelegt ist, den Tongenerator (2) derart anzusteuern, dass dieser für den paarweisen Tonvergleich Töne vorspielt, die innerhalb eines vorgegebenen Frequenzbereichs um die Frequenz des als Ergebnis der Ähnlichkeitsmessung ermittelten Tons, dessen Frequenz der Frequenz des mindestens einen dominanten Tons des Ohrgeräuschs gemäß der Ähnlichkeitsmessung am ähnlichsten ist, liegen.

2. Vorrichtung (1) nach Anspruch 1, wobei die Steuer- und Analyseeinheit (4) dazu ausgelegt ist, die Untersuchungen der Ähnlichkeitsmessung und des paarweisen Tonvergleichs in einer vorgegebenen Reihenfolge durchzuführen, wobei das Ergebnis der zuerst durchgeführten Untersuchung als Ausgangswert für die nachfolgende Untersuchung verwendet wird.

3. Vorrichtung (1) nach Anspruch 2, wobei für die Ähnlichkeitsmessung und den paarweisen Tonvergleich die Töne, welche der Tongenerator (2) vorspielt, aus jeweils vorgegebenen Tönen ausgewählt werden und der Frequenzabstand von benachbarten, vorgegebenen Tönen für die zuerst durchgeführte Untersuchung größer ist als der Frequenzabstand von benachbarten, vorgegebenen Tönen für die nachfolgende Untersuchung.

4. Vorrichtung (1) nach einem der vorhergehenden Ansprüche, wobei die Steuer- und Analyseeinheit (4) dazu ausgelegt ist, als Ergebnis des paarweisen Tonvergleichs mindestens einen Ton zu ermitteln, dessen Frequenz der Frequenz des mindestens einen dominanten Tons des Ohrgeräuschs gemäß dem paarweisen Tonvergleich am ähnlichsten ist.

5. Vorrichtung (1) nach einem der vorhergehenden Ansprüche, wobei die Steuer- und Analyseeinheit (4) beim paarweisen Tonvergleich diejenigen Töne der vorgespielten Paare von Tönen auswählt, die gemäß der Bewertung des Patienten dem mindestens einen dominanten Ton des Ohrgeräuschs ähnlicher sind, und nachfolgende Paare von Tönen aus den ausgewählten Tönen zusammenstellt.

6. Vorrichtung (1) nach einem der vorhergehenden Ansprüche, wobei bei der Ähnlichkeitsmessung und/oder dem paarweisen Tonvergleich die Ähnlichkeit der Töne vom Patienten mittels eines psychophysischen Ähnlichkeitsmaßes, insbesondere hinsichtlich Tonhöhe und/oder Lautheit und/oder Klangcharakter, bewertet wird.

7. Vorrichtung (1) nach einem der vorhergehenden Ansprüche, wobei ein jeweiliger Ton, der dem Patienten für die Ähnlichkeitsmessung vorgespielt wird, von der von dem Patienten vorgenommenen Bewertung eines zuvor vorgespielten Tons abhängt, und/oder wobei ein jeweiliges Paar von Tönen, die dem Patienten für den paarweisen Vergleich vorgespielt werden, von der von dem Patienten vorgenommenen Bewertung eines zuvor vorgespielten Paars von Tönen abhängt.

8. Vorrichtung (1) nach einem der vorhergehenden Ansprüche, wobei die Steuereinheit (4) den Tongenerator (2) derart ansteuert, dass dieser mindestens einen Ton vorspielt, und der Patient die Lautstärke des mindestens einen Tons über die Eingabeeinheit (3) derart einstellt, dass die Lautstärke des mindestens einen Tons der von dem Patienten wahrgenommenen Lautstärke des mindestens einen dominanten Tons des Ohrgeräuschs entspricht, wobei die Steuereinheit (4) den Tongenerator (2) ferner derart ansteuert, dass die Lautstärke der für die Ähnlichkeitsmessung und/oder den paarweisen Tonvergleich vorgespielten Töne an die von dem Patienten eingestellte Lautstärke angepasst ist.

9. Vorrichtung (1) nach einem der vorhergehenden Ansprüche, wobei die Steuer- und Analyseeinheit (4) dazu ausgelegt ist, eine grobe Abschätzung der Frequenz des mindestens einen dominanten Tons des von dem Patienten wahrgenommenen Ohrgeräuschs durchzuführen, wozu die Steuereinheit (4) den Tongenerator (2) derart ansteuert, dass dieser nacheinander Töne vorspielt, und die Steuer- und Analyseeinheit (4) nach dem Vorspielen eines jeweiligen Tons die in die Eingabeeinheit (3) eingegebene Bewertung des Patienten entgegennimmt, ob die Frequenz des Tons größer oder kleiner als die Frequenz des mindestens einen dominanten Tons des Ohrgeräuschs ist.

10. Vorrichtung (1) nach Anspruch 9, wobei die Steuer- und Analyseeinheit (4) als Ergebnis der groben Abschätzung der Frequenz des mindestens einen dominanten Tons des von dem Patienten wahrgenommenen Ohrgeräuschs einen Frequenzbereich ermittelt, und die Steuer- und Analyseeinheit (4) für die Ähnlichkeitsmessung und/oder den paarweisen Tonvergleich den Tongenerator (2) derart ansteuert, dass dieser Töne oder Paare von Tönen aus dem ermittelten Frequenzbereich vorspielt.

11. Verfahren zur Ermittlung der Frequenz von mindestens einem dominanten Ton eines von einem Patienten wahrgenommenen Ohrgeräuschs, wobei
- eine Ähnlichkeitsmessung durchgeführt wird, bei welcher dem Patienten nacheinander Töne vorgespielt werden und der Patient nach dem Vorspielen eines jeweiligen Tons die Ähnlichkeit des Tons zu dem mindestens einen dominanten Ton des Ohrgeräuschs bewertet,
- ein paarweiser Tonvergleich durchgeführt wird, bei welchem dem Patienten nacheinander Paare von Tönen vorgespielt werden und der Patient nach dem Vorspielen eines jeweiligen Paars bewertet, welcher Ton des Paars dem mindestens einen dominanten Ton des Ohrgeräuschs ähnlicher ist, **dadurch gekennzeichnet, dass**
- die Ergebnisse der Ähnlichkeitsmessung und des paarweisen Tonvergleichs kombiniert werden und daraus mindestens ein Ton ermittelt wird, dessen Frequenz der Frequenz des mindestens einen dominanten Tons des Ohrgeräuschs am ähnlichsten ist,
- als Ergebnis der Ähnlichkeitsmessung ein Ton ermittelt wird, dessen Frequenz der Frequenz des mindestens einen dominanten Tons des Ohrgeräuschs gemäß der Ähnlichkeitsmessung am ähnlichsten ist, und
- für den paarweisen Tonvergleich Töne vorgespielt werden, die innerhalb eines vorgegebenen Frequenzbereichs um die Frequenz des als Ergebnis der Ähnlichkeitsmessung ermittelten Tons, dessen Frequenz der Frequenz des mindestens einen dominanten Tons des Ohrgeräuschs gemäß der Ähnlichkeitsmessung am ähnlichsten ist, liegen.

12. Software zum Ausführen in einem Datenverarbeitungssystem einer Vorrichtung gemäss Anspruch 1, wobei die Software
- Steuersignale zum Ansteuern eines Tongenerators (2) zum Vorspielen von Tönen erzeugt,
- von einer Eingabeeinheit (3) Bewertungen der vorgespielten Töne durch einen Patienten entgegennimmt,
- den Tongenerator (2) für eine Ähnlichkeitsmessung derart ansteuert, dass dieser nacheinander Töne vorspielt, und nach dem Vorspielen eines jeweiligen Tons von der Eingabeeinheit (3) eine Bewertung des Patienten bezüglich der Ähnlichkeit des Tons zu mindestens einem dominanten Ton eines von dem Patienten wahrgenommenen Ohrgeräuschs entgegennimmt,
- den Tongenerator (2) für einen paarweisen Tonvergleich derart ansteuert, dass dieser nacheinander Paare von Tönen vorspielt, und nach dem Vorspielen eines jeweiligen Paars von der Eingabeeinheit (3) eine Bewertung des Patienten entgegennimmt, welcher Ton des Paars dem mindestens einen dominanten Ton des Ohrgeräuschs ähnlicher ist, **dadurch gekennzeichnet, dass**
- die Ergebnisse der Ähnlichkeitsmessung und des paarweisen Tonvergleichs kombiniert und daraus mindestens einen Ton ermittelt, dessen Frequenz der Frequenz des mindestens einen dominanten Tons des Ohrgeräuschs am ähnlichsten ist,
- als Ergebnis der Ähnlichkeitsmessung einen Ton ermittelt, dessen Frequenz der Frequenz des mindestens einen dominanten Tons des Ohrgeräuschs gemäß der Ähnlichkeitsmessung am ähnlichsten ist, und
- den Tongenerator (2) derart ansteuert, dass dieser für den paarweisen Tonvergleich Töne vorspielt, die innerhalb eines vorgegebenen Frequenzbereichs um die Frequenz des als Ergebnis der Ähnlichkeitsmessung ermittelten Tons, dessen Frequenz der Frequenz des mindestens einen dominanten Tons des Ohrgeräuschs gemäß der Ähnlichkeitsmessung am ähnlichsten ist, liegen.

## Claims

1. An apparatus (1) for determining the frequency of at least one dominant sound of a ringing in an ear perceived by a patient, the apparatus comprising
- a sound generator (2) for the playing of sounds,
- an input unit (3) for inputting evaluations of the played sounds by the patient, and
- a control and analysis unit (4) coupled to the sound generator (2) and to the input unit (3), the control and analysis unit being configured to carry out a similarity measurement and a pairwise sound comparison, wherein
- the control and analysis unit (4) controls the sound generator (2) for the similarity measurement in such a manner that this plays sounds one after the other, and the control and analysis unit (4) receives, after the playing of a sound, the evaluation of the patient input into the input unit (3) with respect to the similarity of the respective sound to the at least one dominant sound of the ringing in the ear,
- the control and analysis unit (4) controls the sound generator (2) for the pairwise sound comparison in such a manner that this plays pairs of sounds one after the other, and the control and analysis unit (4) receives, after the playing of a pair, an evaluation of the patient input into the input unit (3) about which sound of the respective pair is more similar to the at least one dominant sound of the ringing in the ear,
**characterized in that**
- the control and analysis unit (4) is configured to combine results of the similarity measurement and of the pairwise sound comparison and, based thereon, determine at least one sound having a frequency that is most similar to the frequency of the at least one dominant sound of the ringing in the ear,
- the control and analysis unit (4) is configured to determine, as a result of the similarity measurement, a sound having a frequency that is most similar to the frequency of the at least one dominant sound of the ringing in the ear in accordance with the similarity measurement, and
- the control and analysis unit (4) is configured to control the sound generator (2) in such a manner that this generates sounds for the pairwise sound comparison that lie within a predefined frequency range around the frequency of the sound determined as a result of the similarity measurement, which has the frequency that is most similar to the frequency of the at least one dominant sound of the ringing in the ear in accordance with the similarity measurement.

2. The apparatus (1) in accordance with claim 1, wherein the control and analysis unit is configured to perform analyses of the similarity measurement and the pairwise sound comparison in a predefined sequence, wherein the result of the analysis carried out first is used as a starting value for a subsequent analysis.

3. The apparatus (1) in accordance with claim 2, wherein the sounds that the sound generator (2) plays for the similarity measurement and the pairwise sound comparison are selected from respectively predefined sounds and a frequency spacing of adjacent predefined sounds is larger for the analysis carried out first than the frequency spacing of adjacent predefined sounds for the subsequent analysis.

4. The apparatus (1) in accordance with any one of the preceding claims, wherein the control and analysis unit (4) is configured to determine at least one sound as a result of the pairwise sound comparison having a frequency that is most similar to the frequency of the at least one dominant sound of the ringing in the ear in accordance with the pairwise sound comparison.

5. The apparatus (1) in accordance with any one of the preceding claims, wherein the control and analysis unit (4) is configured to select, during the pairwise sound comparing, those sounds of the played pairs of sounds that are more similar to the at least one dominant sound of the ringing in the ear in accordance with the evaluation of the patient, and the control and analysis unit (4) combines the selected sounds for the subsequent pairs of sounds.

6. The apparatus (1) in accordance with any one of the preceding claims, wherein, for the similarity measurement and/or the pairwise sound comparison, the similarity of the sounds is evaluated by the patient based on a psychophysical degree of similarity, in particular with respect to pitch and/or volume and/or timbre.

7. The apparatus (1) in accordance with any one of the preceding claims, wherein a respective sound that is played to the patient for the similarity measurement depends on the evaluation carried out by the patient of a previously played sound, and/or wherein a respective pair of sounds that is played to the patient for the pairwise comparison depends on the evaluation carried out by the patient of a previously played pair of sounds.

8. The apparatus (1) in accordance with any one of the preceding claims, wherein the control unit (4) is configured to control the sound generator (2) to play at least one sound and the patient sets a volume level of the at least one sound via the input unit such that the set volume level of the at least one sound corresponds to a volume level of the at least one dominant sound of the ringing in the ear perceived by the patient, wherein the control unit (4) is further configured to control the sound generator (2) such that the volume level of the sounds played for the similarity measurement and/or the pairwise sound comparison is matched to the volume level set by the patient.

9. The apparatus (1) in accordance with any one of the preceding claims, wherein the control and analysis unit (4) is configured to perform a coarse estimate of the frequency of the at least one dominant sound of the ringing in the ear perceived by the patient, for which purpose the control unit (4) controls the sound generator (2) such that the sound generator (2) plays the sounds one after the other and the control and analysis unit (4) receives, after the playing of a sound, the evaluation input by the patient into the input unit (3) about whether the frequency of the respective sound is larger or smaller than the frequency of the at least one dominant sound of the ringing in the ear.

10. The apparatus in accordance with claim 9, wherein, based on the coarse estimate of the frequency of the at least one dominant sound of the ringing in the ear perceived by the patient, the control and analysis unit (4) is configured to determine a frequency range and control the sound generator (2) for the similarity measurement and/or the pairwise sound comparison such that the sound generator (2) plays the sounds or pairs of sounds from the determined frequency range.

11. A method of determining a frequency of at least one dominant sound of a ringing in an ear perceived by a patient, wherein:
- a similarity measurement is performed, in which sounds are played one after the other for the patient and the patient evaluates, after the playing of a sound, a similarity of the respective sound to the at least one dominant sound of the ringing in the ear;
- a pairwise sound comparison is performed, in which pairs of sounds are played one after the other for the patient and the patient evaluates, after the playing of a pair, which sound of the respective pair is more similar to the at least one dominant sound of the ringing in the ear;
- results of the similarity measurement and the pairwise sound comparison are combined and based thereon at least one sound is determined, which has a frequency that is most similar to the frequency of the at least one dominant sound of the ringing in the ear;
- as a result of the similarity measurement a sound is determined, which has a frequency that is most similar to the frequency of the at least one dominant sound of the ringing in the ear in accordance with the similarity measurement; and
- sounds are played for the pairwise sound comparison, the sounds lying within a predefined frequency range around the frequency of the sound determined as a result of the similarity measurement, which has the frequency that is most similar to the frequency of the at least one dominant sound of the ringing in the ear in accordance with the similarity measurement.

12. Software for execution in a data processing system of an apparatus according to claim 1, wherein the software:
- generates control signals for controlling a sound generator (2) for the playing of sounds,
- receives evaluations of the played sounds by the patient from an input unit (3),
- controls the sound generator (2) for a similarity measurement in such a manner that this plays sounds one after the other, and, after the playing of a sound, receives the evaluation of the patient from the input unit (3) with respect to the similarity of the respective sound to the at least one dominant sound of the ringing in the ear,
- controls the sound generator (2) for a pairwise sound comparison in such a manner that this plays pairs of sounds one after the other, and, after the playing of a pair, receives an evaluation of the patient from the input unit (3) about which sound of the respective pair is more similar to the at least one dominant sound of the ringing in the ear,
**characterized in that**
- it combines results of the similarity measurement and of the pairwise sound comparison and, based thereon, determines at least one sound having a frequency that is most similar to the frequency of the at least one dominant sound of the ringing in the ear,
- it determines, as a result of the similarity measurement, a sound having a frequency that is most similar to the frequency of the at least one dominant sound of the ringing in the ear in accordance with the similarity measurement, and
- it controls the sound generator (2) in such a manner that this generates sounds for the pairwise sound comparison that lie within a predefined frequency range around the frequency of the sound determined as a result of the similarity measurement, which has the frequency that is most similar to the frequency of the at least one dominant sound of the ringing in the ear in accordance with the similarity measurement.

## Revendications

1. Dispositif (1) pour la détermination de la fréquence d'au moins un son dominant d'un acouphène perçu par un patient, comprenant
- un générateur de sons (2) pour le jeu de sons,
- un module de saisie (3) pour la saisie d'évaluations des sons joués par le patient, et
- un module de commande et d'analyse (4) couplé au générateur de sons (2) et au module de saisie (3) qui est conçu pour effectuer une mesure de similarité et une comparaison de sons par paire, dans lequel
- le module de commande et d'analyse (4) commande pour la mesure de similarité le générateur de sons (2) de telle sorte que celui-ci joue des sons successivement, et le module de commande et d'analyse (4) réceptionne après le jeu d'un son respectif l'évaluation du patient saisie dans le module de saisie (3) concernant la similarité du son à l'au moins un son dominant de l'acouphène,
- le module de commande et d'analyse (4) commande pour la comparaison de sons par paire le générateur de sons (2) de telle sorte que celui-ci joue des paires de sons successivement, et le module de commande et d'analyse (4) réceptionne après le jeu d'une paire respective l'évaluation du patient saisie dans le module de saisie (3) du son de le paire qui est davantage similaire à l'au moins un son dominant de l'acouphène, **caractérisé en ce que**
- le module de commande et d'analyse (4) combine les résultats de la mesure de similarité et de la comparaison de sons par paire et détermine à partir de ceux-ci au moins un son dont la fréquence est la plus similaire à la fréquence de l'au moins un son dominant de l'acouphène,
- le module de commande et d'analyse (4) est conçu pour déterminer en tant que résultat de la mesure de similarité un son dont la fréquence est la plus similaire à la fréquence de l'au moins un son dominant de l'acouphène selon la mesure de similarité, et
- le module de commande et d'analyse (4) est conçu pour commander le générateur de sons (2) de telle sorte que celui-ci joue pour la comparaison de sons par paire des sons qui se situent au sein d'une plage de fréquences prédéfinie autour de la fréquence du son déterminé en tant que résultat de la mesure de similarité dont la fréquence est la plus similaire à la fréquence de l'au moins un son dominant de l'acouphène selon la mesure de similarité.

2. Dispositif (1) selon la revendication 1, dans lequel le module de commande et d'analyse (4) est conçu pour effectuer les examens de la mesure de similarité et de la comparaison de sons par paire dans un ordre prédéfini, dans lequel le résultat de l'examen effectué en premier est utilisé en tant que valeur de départ pour l'examen subséquent.

3. Dispositif (1) selon la revendication 2, dans lequel les sons que joue le générateur de sons (2) sont sélectionnés pour la mesure de similarité et la comparaison de sons par paire parmi des sons prédéfinis respectivement et l'écart de fréquence de sons prédéfinis voisins pour l'examen effectué en premier est supérieur à l'écart de fréquence de sons prédéfinis voisins pour l'examen subséquent.

4. Dispositif (1) selon une des revendications précédentes, dans lequel le module de commande et d'analyse (4) est conçu pour déterminer en tant que résultat de la comparaison de sons par paire au moins un son dont la fréquence est la plus similaire à la fréquence de l'au moins un son dominant de l'acouphène selon la comparaison de sons par paire.

5. Dispositif (1) selon une des revendications précédentes, dans lequel le module de commande et d'analyse (4) sélectionne lors de la comparaison de sons par paire les sons des paires jouées de sons qui sont davantage similaires à l'au moins un son dominant de l'acouphène selon l'évaluation du patient, et compose des paires subséquentes de sons à partir des sons sélectionnés.

6. Dispositif (1) selon une des revendications précédentes, dans lequel la similarité des sons est évaluée par le patient lors de la mesure de similarité et/ou la comparaison de sons par paire au moyen d'un degré de similarité psychophysique, notamment en ce qui concerne la hauteur de son et/ou la sonie et/ou le caractère de sonorité.

7. Dispositif (1) selon une des revendications précédentes, dans lequel un son respectif qui est joué au patient pour la mesure de similarité dépend de l'évaluation effectuée par le patient d'un son joué au préalable, et/ou dans lequel une paire respective de sons qui sont joués au patient pour la comparaison par paire dépend de l'évaluation effectuée par le patient d'une paire de sons jouée au préalable.

8. Dispositif (1) selon une des revendications précédentes, dans lequel le module de commande (4) commande le générateur de sons (2) de telle sorte que celui-ci joue au moins un son, et le patient règle le volume de l'au moins un son par le biais du module de saisie (3) de telle sorte que le volume de l'au moins un son correspond au volume perçu par le patient de l'au moins un son dominant de l'acouphène, dans lequel le module de commande (4) commande en outre le générateur de sons (2) de telle sorte que le volume des sons joués pour la mesure de similarité et/ou la comparaison de sons par paire est adapté au volume réglé par le patient.

9. Dispositif (1) selon une des revendications précédentes, dans lequel le module de commande et d'analyse (4) est conçu pour effectuer une estimation grossière de la fréquence de l'au moins un son dominant de l'acouphène perçu par le patient, ce pour quoi le module de commande (4) commande le générateur de sons (2) de telle sorte que celui-ci joue des sons successivement, et le module de commande et d'analyse (4) réceptionne après le jeu d'un son respectif l'évaluation du patient saisie dans le module de saisie (3) du fait que la fréquence du son soit supérieure ou inférieure à la fréquence de l'au moins un son dominant de l'acouphène.

10. Dispositif (1) selon la revendication 9, dans lequel le module de commande et d'analyse (4) détermine une plage de fréquences en tant que résultat de l'estimation grossière de la fréquence de l'au moins un son dominant de l'acouphène perçu par le patient, et le module de commande et d'analyse (4) commande pour la mesure de similarité et/ou la comparaison de sons par paire le générateur de sons (2) de telle sorte que celui-ci joue des sons ou paires de sons provenant de la plage de fréquences déterminée.

11. Procédé pour la détermination de la fréquence d'au moins un son dominant d'un acouphène perçu par un patient, dans lequel
- une mesure de similarité est effectuée, lors de laquelle des sons sont joués successivement au patient et le patient évalue après le jeu d'un son respectif la similarité du son à l'au moins un son dominant de l'acouphène,
- une comparaison de sons par paire est effectuée, lors de laquelle des paires de sons sont jouées successivement au patient et le patient évalue après le jeu d'une paire respective quel son de la paire est davantage similaire à l'au moins un son dominant de l'acouphène, **caractérisé en ce que**
- les résultats de la mesure de similarité et de la comparaison de sons par paire sont combinés et au moins un son est déterminé à partir de ceux-ci dont la fréquence est la plus similaire à la fréquence de l'au moins un son dominant de l'acouphène,
- un son dont la fréquence est la plus similaire à la fréquence de l'au moins un son dominant de l'acouphène selon la mesure de similarité est déterminé en tant que résultat de la mesure de similarité, et
- des sons qui se situent au sein d'une plage de fréquences prédéfinie autour de la fréquence du son déterminé en tant que résultat de la mesure de similarité dont la fréquence est la plus similaire à la fréquence de l'au moins un son dominant de l'acouphène selon la mesure de similarité sont joués pour la comparaison de sons par paire.

12. Logiciel pour l'exécution dans un système de traitement de données d'un dispositif selon la revendication 1, dans lequel le logiciel
- génère des signaux de commande pour la commande d'un générateur de sons (2) pour le jeu de sons,
- réceptionne des évaluations des sons joués par un patient par un module de saisie (3),
- commande le générateur de sons (2) pour une mesure de similarité de telle sorte que celui-ci joue des sons successivement, et réceptionne après le jeu d'un son respectif par le module de saisie (3) une évaluation du patient concernant la similarité du son à au moins un son dominant d'un acouphène perçu par le patient,
- commande le générateur de sons (2) pour une comparaison de sons par paire de telle sorte que celui-ci joue des paires de sons successivement, et réceptionne après le jeu d'une paire respective par le module de saisie (3) une évaluation du patient du son de la paire qui est davantage similaire à l'au moins un son dominant de l'acouphène, **caractérisé en ce que**
- combine les résultats de la mesure de similarité et de la comparaison de sons par paire et détermine au moins un son à partir de ceux-ci dont la fréquence est la plus similaire à la fréquence de l'au moins un son dominant de l'acouphène,
- détermine un son dont la fréquence est la plus similaire à la fréquence de l'au moins un son dominant de l'acouphène selon la mesure de similarité en tant que résultat de la mesure de similarité, et
- commande le générateur de sons (2) de telle sorte que celui-ci joue pour la comparaison de sons par paire des sons qui se situent au sein d'une plage de fréquences prédéfinie autour de la fréquence du son déterminé en tant que résultat de la mesure de similarité dont la fréquence est la plus similaire à la fréquence de l'au moins un son dominant de l'acouphène selon la mesure de similarité.
